# DEMANDE DE BREVET EUROPEEN

(11) **EP 1 277 458 A1**
(43) Date de publication de la demande: **22.01.2003**
(21) Numéro de dépôt: 02291753.8
(22) Date de dépôt: 11.07.2002
(51) Int. Cl.: A61K 7/035, A61K 7/48

(54) **Composition cosmétique pulvérulente**

(30) Priorité: 20.07.2001 FR 0109768
(71) Demandeur: L'OREAL, 75008 Paris (FR)
(72) Inventeur: Jager Lezer, Nathalie, 91000 Verrieres-Le-Buisson (FR)
(74) Mandataire: Kromer, Christophe

(57) **Abrégé**

L'invention concerne une composition cosmétique, notamment de maquillage, pulvérulente comprenant au moins une phase particulaire et au moins une phase grasse, caractérisée en ce qu'elle comprend en outre un agent gélifiant de type élastomère de silicone à propriété tensioactive.

## Description

La présente invention a pour objet une composition cosmétique, notamment de maquillage, sous forme de poudre, par exemple libre, compacte ou pressée, comprenant un agent gélifiant particulier.

Les poudres de maquillage comprennent généralement, d'une part une phase particulaire comportant notamment des pigments et des charges, et d'autre part une phase grasse au titre de liant comprenant des corps gras, destinée à conférer au produit fini une certaine cohésion, à donner une douceur et une propriété émolliente au produit de maquillage et à favoriser son adhérence sur la peau. Elles peuvent aussi comprendre une phase hydrophile, par exemple aqueuse, ce qui leur donne des propriétés de fraîcheur à l'application.

La formulation des agents liants dans les poudres, en particulier dans les poudres compactes, soulève de nombreuses difficultés car la composition finale doit être suffisamment homogène et compacte pour présenter une bonne aptitude au prélèvement et pour éviter par ailleurs une fragmentation provoquée notamment par des chocs.

Les poudres de maquillage sont donc des produits constitués généralement d'un très fort taux de composés pulvérulents et de corps gras. Ces produits peuvent ainsi donner des sensations de tiraillement ou un effet desséchant lorsqu'ils sont appliqués sur la peau.

L'effet de matité est particulièrement recherché pour les utilisateurs à peaux mixtes ou grasses, ainsi que sous les climats chauds et humides. Les charges matifiantes utilisées dans les poudres de maquillage sont le plus souvent des charges absorbantes, en particulier du sébum et d'huile excédentaire apportée par la composition, comme le talc, la silice, le kaolin, la poudre de nylon ou encore des charges présentant des propriétés optiques de diffusion de la lumière, propriétés connues sous le nom d'effet « soft focus ». Une composition présentant des propriétés de « soft-focus » est telle que, lorsqu'on l'applique sur la peau, elle donne un effet flou qui camoufle les microreliefs de la peau. Ces charges ont tendance à dessécher la peau, à marquer (c'est-à-dire à rendre plus visible, à accentuer, à faire apparaître) le relief cutané, en particulier les rides, les pores, accentuant ainsi les imperfections locales. De plus, les charges absorbantes confèrent à la peau un aspect poudreux peu naturel et ont un effet matifiant peu durable dans le temps.

Il subsiste donc un besoin de composition pulvérulente mate, confortable et qui ne marque pas le relief cutané, en particulier des zones sèches du visage.

La Demanderesse a trouvé de manière inattendue que l'utilisation d'un agent gélifiant de type élastomère de silicone à propriété tensioactive au sein d'une composition pulvérulente permet l'obtention d'une poudre qui présente d'excellentes propriétés cosmétiques et/ou optiques, surtout en terme de matification, mais aussi en terme d'effet « soft-focus ».

L'invention a donc pour objet une composition cosmétique sous forme de poudre comprenant une phase particulaire et une phase grasse, caractérisée par le fait qu'elle comprend en outre au moins un agent gélifiant de type élastomère de silicone à propriété tensioactive et la phase particulaire étant présente en une teneur comprise entre 50 % et 98 % en poids, par rapport au poids total de la composition.

De préférence, l'agent gélifiant est dispersé dans une phase grasse liquide, par exemple une huile siliconée, hydrocarbonée, de préférence une huile siliconée.

On obtient ainsi une poudre cosmétique présentant une bonne cohésion et que l'on peut utiliser facilement pour se maquiller.

Les compositions selon l'invention présentent l'avantage de matifier les matières kératiniques sur lesquelles elles sont appliquées.

Les compositions ainsi obtenues présentent avantageusement une excellente dispersion des pigments. La composition obtenue est très homogène et elle le reste même après application sur la peau, et ce généralement pendant plusieurs heures.

Les compositions selon l'invention présentent également d'excellentes propriétés cosmétiques : elles adhèrent suffisamment à la peau mais pas trop, elles sont très douces, elles s'appliquent facilement.

La présente invention a encore pour objet un procédé cosmétique de maquillage ou de soin des matières kératiniques des êtres humains, en particulier de la peau, notamment du visage et du corps, comprenant l'application sur ces matières de la composition telle que définie ci-dessus.

Par matières kératiniques, on entend selon la présente invention, la peau, les ongles, les cheveux, les cils et sourcils, les muqueuses (intérieurs des paupières inférieures) et les semi-muqueuses (lèvres), et toute autre zone cutanée du corps et du visage.

La présente invention a encore pour objet l'utilisation d'au moins un agent gélifiant de type élastomère de silicone à propriété tensioactive dans une composition cosmétique pulvérulente, dans le but d'apporter de la matité aux matières kératiniques sur lesquelles est appliquée ladite composition.

La présente invention a également pour objet l'utilisation d'au moins un agent gélifiant de type élastomère de silicone à propriété tensioactive, dans une composition cosmétique pulvérulente, dans le but d'améliorer le développement de la couleur au sein de ladite composition.

Ainsi, il est possible, grâce à la présente invention, de réaliser des compositions comprenant moins de pigments que les compositions de l'art antérieur tout en obtenant la même intensité en couleur. De même, en utilisant le même taux de pigments qu'une composition de l'art antérieur, l'utilisation de l'agent gélifiant selon l'invention permet d'obtenir une composition dont la couleur est beaucoup plus vivace, plus soutenue voire intense, plus développée.

De plus, la composition selon l'invention est une composition correctrice du teint, c'est-à-dire qu'elle sert à matifier la peau ou les lèvres et/ou à camoufler les imperfections de la peau et/ou des lèvres. L'invention concerne donc aussi l'utilisation d'un agent gélifiant de type élastomère de silicone à propriété tensioactive, dans une composition ou pour la fabrication d'une composition de soin ou de maquillage pour matifier la peau ou les lèvres et/ou camoufler les imperfections de la peau et/ou des lèvres.

Par imperfections, on entend tout défaut de la peau comme le microrelief (pores, rides, ridules), les tâches et les points noirs, les microvaisseaux sanguins.

Les compositions selon l'invention trouvent notamment une application particulièrement intéressante dans le domaine du maquillage et/ou du soin de la peau. Ainsi, l'invention trouve une application toute particulière dans le domaine des produits de maquillage du visage et du corps. Par suite la composition selon l'invention se présente avantageusement sous la forme de fard à paupières, de fard à joues, de fard à sourcils, de poudre du visage et/ou du corps, de produit anticernes, de produit matifiant, ou de produit de maquillage du corps.

D'autres caractéristiques, aspects et avantages de la présente invention apparaîtront à la lecture de la description détaillée qui va suivre.

Les compositions selon l'invention comprennent un agent gélifiant de type élastomère de silicone à propriété tensioactive et qui peut représenter jusqu'à 10% en poids, de préférence de 0,3 % à 10% en poids, et préférentiellement encore de 0,6 % à 6% en poids, par rapport au poids total de la composition.

Par « élastomère » on entend un matériau souple, déformable ayant des propriétés viscoélastiques et notamment la consistance d'une éponge ou d'une sphère souple. Son module d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Avantageusement, l'agent gélifiant selon l'invention est un organopolysiloxane élastomérique solide réticulé sous forme de particules et comprend au moins un groupement oxyalkyléné et plus spécialement oxyéthyléné (OE), par exemple de 1 à 40 motifs oxyalkylénés, de préférence de 1 à 20, de façon encore plus préférée de 10 à 20, et de façon encore plus préférée de 12 à 18 et mieux de 12 à 20, pouvant former des chaînes polyoxyalkylènes et notamment polyoxyéthylènes. De préférence l'organopolysiloxane élastomérique ne comporte que des groupements oxyéthylénés comme groupement oxyalkylénés. De tels groupements confèrent avantageusement à l'agent gélifiant selon l'invention une propriété tensioactive. Ces groupements peuvent être pendants, en bout de chaîne et/ou destinés à lier deux parties de la structure siliconée. Le nombre de silicium portant ces groupements est avantageusement de 1 à 10, de préférence de 1 à 6.

Dans la présente demande, on entend par groupement oxyalkyléné un groupement ayant de 2 à 4 atomes de carbone, et en particulier un groupement oxyéthyléné ou oxypropyléné.

Bien que l'invention concerne plus spécialement les agents gélifiants à groupement(s) oxyéthyléné(s) (à savoir ne comportant que des groupements oxyéthylénés comme groupements oxyalkylénés), elle peut aussi concerner les agents gélifiants à groupement(s) oxypropyléné(s), c'est-à-dire ne comportant que des groupements oxypropylénés comme groupements oxyalkylénés. Les agents gélifiants peuvent aussi comporter à la fois au moins un groupement oxyéthyléné 1 à 20 (OE) par exemple et au moins un groupement oxypropyléné (OP), 0 à 20 par exemple ; ces agents gélifiants sont aussi appelés des organopolysiloxanes à groupement(s) alkyléthoxy-propyléné(s). De préférence le nombre de groupements oxyéthylénés est supérieur au nombre de groupements oxypropylénés.

Par ailleurs, la structure siliconée formant le squelette polymérique de l'agent gélifiant est avantageusement une structure polydiméthylsiloxane (PDMS) dont éventuellement une partie des groupes méthyle est substituée par des groupements alkyles en C₂ à C₃₀ et de préférence en C₈ à C₂₄, et mieux de C₁₀ à C₂₀ ou phényle, soit en bout de chaîne soit pendants.

Par ailleurs, l' orgapolysiloxane à groupement(s) oxyalkyléné(s) peut comporter un ou plusieurs squelette(s) siliconé(s) relié(s) entre eux par un ou plusieurs groupements oxyalkylénés et de préférence oxyéthylénés tels que définis précédemment. De préférence, il comporte au moins deux squelettes polymériques liés entre eux.

Avantageusement, le (ou les) squelette(s) siliconé(s) des organopolysiloxanes élastomériques de la composition selon l'invention contiennent de 26 à 80 atomes de silicium.

Selon l'invention, l'agent gélifiant peut servir comme émulsifiant d'une phase aqueuse dans une phase grasse liquide.

L'agent gélifiant selon l'invention présente un remarquable pouvoir épaississant de phase grasse liquide et d'émulsification d'une phase aqueuse dans une phase grasse liquide. Il n'est pas desséchant pour la peau et apporte de bonnes propriétés cosmétiques, notamment de douceur et de fraîcheur. Cet agent gélifiant élastomère conduit à des compositions pulvérulentes confortables à l'application, à texture crémeuse, de bon étalement, douces et non collantes au toucher. Ces propriétés cosmétiques sont dues, d'une part à la texture de l'organopolysiloxane qu'est l'agent gélifiant et, d'autre part à leurs propriétés comparables à celles de microéponges piégeant les milieux huileux et en particulier ceux de la composition et ceux sécrétés par la peau.

La propriété de matifier la peau des compositions selon l'invention est remarquable, et attribuée au moins en partie à la présence de ces organopolysiloxanes à groupement(s) oxyalkyléné(s) et notamment oxyéthyléné(s).

Selon un mode de réalisation de l'invention, la composition de l'invention est une émulsion simple ou multiple, à phase continue lipophile, de préférence à phase continue grasse liquide, et est exempte de tensioactif autre que l'agent gélifiant selon l'invention.

La composition selon l'invention est stable, c'est-à-dire qu'elle ne démixe pas à température ambiante pendant au moins 2 mois.

Dans un mode de réalisation préféré de l'invention, l'agent gélifiant selon l'invention est un organopolysiloxane élastomérique partiellement ou totalement réticulé et de structure tridimensionnelle, présent dans une phase grasse liquide, de préférence huileuse. Inclus dans une phase grasse liquide, de préférence huileuse, il se transforme, selon le taux de phase grasse liquide utilisé, d'un produit d'aspect spongieux lorsqu'il est utilisé en présence de faibles teneurs en phase grasse liquide en un gel homogène, en présence de quantités de phase grasse liquide plus élevées. La gélification de la phase grasse liquide par cet élastomère peut être totale ou partielle.

Selon un tel mode de réalisation, l'agent gélifiant élastomère de l'invention est présent dans une phase grasse liquide, notamment sous forme dispersée, et se présente ainsi sous forme de pâte ou de gel.

Par phase grasse liquide, appelée encore phase huileuse, on entend tout corps non aqueux ou mélange de corps non aqueux, liquides à température ambiante (environ 25°C) et pression atmosphérique (environ 1,013. 10⁵ Pa).

L'agent gélifiant selon l'invention peut être choisi parmi les polymères réticulés obtenus par réaction d'addition et de réticulation en milieu non aqueux, en présence d'un catalyseur notamment du type platine, d'au moins :
- (a) un premier organopolysiloxane (i) ayant au moins deux groupes vinyliques en position α-ω de la chaîne siliconée ; et
- (b) un second organopolysiloxane (ii) ayant au moins un atome d'hydrogène lié à un atome de silicium par molécule et au moins groupement oxyalkylène, notamment oxyéthyléné.

En particulier, l'organopolysiloxane (i) est choisi parmi les polydiméthylsiloxanes (PDMS) et est plus spécifiquement un α-ω-diméthylvinyl polydiméthylsiloxane. L'organo-polysiloxane (ii) est choisi notamment parmi les polydiméthylsiloxanes comportant un ou plusieurs atome(s) d'hydrogène, lié chacun à un atome de silicium, et un ou plusieurs groupements oxyalkylénés, de préférence oxyéthylénés, et éventuellement un ou plusieurs groupements oxypropylénés, liés à un atome de silicium via un radical alkylène ayant de 1 à 22 atomes de carbone.

Eventuellement les chaînes silicones des premiers et seconds organopolysiloxanes (i) et (ii) comportent des chaînes pendantes alkyle en C₁ à C₆ et/ou des chaînes aryle.

Comme indiqué précédemment, l'organopolysiloxane elastomère peut se présenter dans une phase grasse liquide avec laquelle il forme un gel anhydre. Ce gel peut être notamment obtenu comme suit :
- (a) mélange du premier organopolysiloxane (i) et du second organopolysiloxane (ii);
- (b) ajout de la phase grasse liquide, de préférence huileuse, au mélange de l'étape (a) ; et
- (c) polymérisation du premier organopolysiloxane (i) et du second organopolysiloxane (ii) en phase grasse liquide, de préférence huileuse, en présence d'un catalyseur de platine.

La phase huileuse qui peut être utilisée lors de la fabrication du gel anhydre contient une ou plusieurs huiles liquides à température ambiante (25°C) choisies parmi les huiles hydrocarbonées et/ou les huiles siliconées. Avantageusement, la phase huileuse est une phase liquide siliconée, contenant une ou plusieurs huiles choisies parmi les PDMS à chaîne linéaire ou cyclique liquides à température ambiante comportant éventuellement une chaîne alkyle ou aryle pendante ou en bout de chaîne, la chaîne alkyle ayant de 1 à 6 atomes de carbone.

Par huiles hydrocarbonées, on entend des huiles contenant majoritairement des atomes de carbone et des atomes d'hydrogène et en particulier des chaînes alkyle ou alcényle comme les alcanes, les alcènes. Ces huiles peuvent, en outre, comporter un ou plusieurs groupes ester, éther, hydroxyle ou carboxylique.

L'élastomère de silicone à propriété tensioactive peut être présent dans la composition en une teneur, en matière active, allant de 0,3 % à 10% en poids, de préférence allant de 0,6 % à 6%, en poids par rapport au poids total de la composition.

L'agent gélifiant selon l'invention est en particulier un organopolysiloxane obtenu selon le mode opératoire des exemples 3, 4 et 8 du document US-A-5412004 et des exemples du document US-A-5811487.

L'élastomère de silicone à propriéyté tensioactive de l'invention est par exemple choisi parmi ceux commercialisés sous les dénominations KSG 21, KSG 31, KSG 32, KSG 41, KSG 50 par la société SHIN ETSU, DC 9010, DC 9011 par la société DOW CORNING, ou bien encore peut être le produit de l'exemple 3 du brevet US-A-5412004.

Le produit de l'exemple 3 du document US-A-5412004 se présente sous la forme d'un gel pâteux contenant environ 33 % en poids d'organopolysiloxane réticulé à groupement(s) oxyéthyléné(s) et environ 67 % de PDMS 6cSt. L'organopolysiloxane contient environ 18 % du poids total du polymère d'oxyde d'éthylène.

Avantageusement, l'agent gélifiant de l'invention, en mélange avec de l'huile, par exemple à 33 % en poids dans le PDM 6 cst, présente les propriétés suivantes :
- l'agent gélifiant a un comportement rhéofluidifiant plastique de viscosité dynamique sous faible cisaillement voisin de 10⁻³s⁻¹ ou 10⁻⁴s⁻¹, allant de 2.10⁶P à 4.10⁶P et une viscosité dynamique de 15 à 50 P (1,5 à 5 Pa.s) pour une vitesse de cisaillement de 200s⁻¹ à t = 10 min, mesurée avec un rhéomètre à contrainte imposée, RS 75 (Haake) à 25°C en géométrie cône/plan ; caractéristique du cône : 20mm de diamètre, 1° d'angle et 40 µm d'entrefer.
- l'agent gélifiant a, en outre, un comportement visco-élastique à 1Hz avec un caractère élastique dominant aux faibles valeurs de la contrainte de cisaillement définie comme suit : 800 Pa < G * ₚₗₐₜₑₐᵤ < 2 500 Pa avec δ ₚₗₐₜₑₐᵤ voisin de 10°, G* ₚₗₐₜₑₐᵤ représentant la consistance et δ ₚₗₐₜₑₐᵤ représentant l'élasticité.
- l'agent gélifiant présente un point éclair d'environ 160 à 170°C à pression atmosphérique. Pour le gel de l'exemple 3, du document US-A-5412004, la viscosité dynamique est d'environ 45 P (4,5 Pa.s) à la vitesse de cisaillement de 200s⁻¹.

Cet agent gélifiant est stable à température ambiante (25°C) au moins 4 mois (sans exsudation d'huile).

En particulier, les particules d'organopolysiloxane élastomérique (matière active) de cet agent gélifiant ont une taille allant de 0,1 à 500 µm, de préférence de 3 à 200 µm, et de façon encore plus préférée de 3 à 50 µm. Ces particules peuvent être sphériques, plates ou amorphes avec, de préférence, une forme sensiblement sphérique, elliptique ou oblongue.

L'agent gélifiant de l'invention est en particulier un tensioactif de HLB (acronyme anglais de BHL = Balance Hydrophile Lipophile) d'environ 2,5. Il est donc parfaitement adapté à la fabrication d'émulsion eau-dans-huile stable et d'émulsion huile-dans-eau-dans-huile ou eau-dans-huile-dans-eau, stable.

A cet agent gélifiant peut être associée une phase grasse comprenant des corps gras liquides à température ambiante, des cires et/ou des gommes solides à température ambiante, des corps gras pâteux, d'origine animale, végétale, minérale ou synthétique, et leurs mélanges.

La phase grasse peut contenir des produits liquides à température ambiante et en particulier des huiles comme les huiles siliconées, fluorées, fluorosiliconées, hydrocarbonées éventuellement partiellement siliconées. Ces huiles peuvent être volatiles à température ambiante et pression atmosphérique. Par huile volatile, on entend en particulier une huile susceptible de s'évaporer, en moins d'une heure, au contact de la peau ou des lèvres ayant notamment une pression vapeur non nulle, en particulier allant de 0,133 à 3,99. 10⁴ Pa (à température ambiante et pression atmosphérique) et de préférence supérieure à 3,99 Pa.

La phase grasse représente généralement de 1,5 à 49,7 % en poids, de préférence de 1,5 à 40 % en poids, de façon encore plus préférée de 1,5 à 30 % en poids, par rapport au poids total de la composition.

Comme huiles utilisables dans la composition de l'invention, on peut citer notamment :
- les huiles hydrocarbonées d'origine animale telles que le perhydrosqualène ;
- les huiles hydrocarbonées végétales telles que les triglycérides liquides d'acides gras, par exemple les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R¹COOR² dans laquelle R¹ représente le reste d'un acide gras supérieur comportant de 7 à 19 atomes de carbone et R² représente une chaîne hydrocarbonée ramifiée contenant de 3 à 20 atomes de carbone comme par exemple l'huile de Purcellin, le myristate d'isopropyle, des octanoates, décanoates ou ricinoléates d'alcools ou de polyalcools
- les hydrocarbures linéaires ou ramifiés, d'origine minérale ou synthétique tels que les huiles de paraffine volatiles ou non et leurs dérivés, la vaseline, les polydécènes, le polyisobutène hydrogéné tel que le Parléam ;
- les éthers de synthèse de formule R³COR⁴ dans laquelle R³ est un radical alkyle en C₃ à C₁₉ et R⁴ un radical alkyle en C₃ à C₂₀.
- des alcools gras comme l'octyl dodécanol ou l'alcool oléique ;
- les huiles fluorées partiellement hydrocarbonées et/ou siliconées comme les perfluoropolyesters ;
- les huiles siliconées comme les polyméthylsiloxanes à chaîne siliconée linéaire ou cyclique, liquides ou pâteux à température ambiante, les phényl diméthicones, les phényl triméthicones et les polyméthylphénylsiloxanes, les alkylpolydiméthylsiloxanes avec une chaîne alkyle en C₂ à C₂₀.
- leurs mélanges.

De préférence l'huile utilisée selon la présente invention est une huile siliconée.

L'agent gélifiant qui est un gel d'organopolysiloxane à groupement(s) oxyéthyéné(s) permet de structurer ces huiles sous forme d'une texture originale type « flan », exempte de gélifiant huileux qui entraverait le toucher doux/soyeux et agréable de la composition.

Avantageusement, la composition selon l'invention peut contenir au moins une cire et/ou une gomme choisie parmi les gommes et les cires hydrocarbonées, fluorées, siliconées et leurs mélanges, qui peuvent être solides ou semi-solides (sous forme d'une pâte) à température ambiante. Ces cires peuvent être d'origine végétale, minérale, animale et/ou synthétique. En particulier, ces cires présentent une température de fusion supérieure à 25 °C, de préférence supérieure à 45 °C, à pression atmosphérique.

Selon l'invention, une cire est un corps gras lipophile, solide à température ambiante, à changements d'état solide/liquide réversible, ayant une température de fusion pouvant aller jusqu'à 200°C et présentant à l'état solide une organisation cristalline anisotrope. En portant la cire à sa température de fusion, il est possible de la rendre miscible à la phase grasse liquide et de former un mélange homogène microscopiquement, puis en ramenant la température du mélange à la température ambiante, on obtient une cristallisation de la cire dans la phase grasse liquide du mélange.

Les cires peuvent être des cires siliconées c'est-à-dire comportant une structure siliconée et des motifs à une ou plusieurs chaînes alkyle ou alcoxy pendantes et/ou en bout de structure siliconée, ces chaînes étant linéaires ou ramifiées et comportant de 10 à 45 atomes de carbone. Ces cires sont appelées respectivement des alkyldiméthicones et des alcoxydiméthicones. Par ailleurs, ces chaînes alkyle peuvent comporter une ou plusieurs fonctions ester.

Comme autre cires utilisables dans l'invention, on peut citer les cires d'origine animale comme la lanoline, la cire d'abeilles ; les cires d'origine végétale telles que la cire de Carnauba ou de Candellila ; les cires d'origine minérale, par exemple de paraffine, de lignite ou les cires microcristallines, la cérésine ou l'ozokérite ; les cires synthétiques comme les cires de polyéthylène.

Ces corps gras peuvent être choisis de manière variée par l'homme du métier afin de préparer une composition ayant les propriétés, par exemple de consistance ou de texture, souhaitées.

En particulier, la présence de cires permet d'assurer une bonne résistance mécanique, notamment lorsque la composition se présente sous la forme d'un produit pressé ou coulé.

Les compositions selon l'invention comprennent également une phase particulaire qui peut comprendre des pigments et/ou des nacres et/ou des charges et/ou des paillettes habituellement utilisés dans les compositions cosmétiques ou connus de l'homme du métier pour remplir un tel usage, et/ou leurs mélanges. Par phase particulaire on entend une phase comprenant des composants sous forme de particules, qui peuvent être de différentes tailles et/ou de différentes natures, et constituée pratiquement essentiellement de telles particules. Par phase particulaire, il faut aussi comprendre un ensemble de particules de toute forme ( par exemple de forme plaquettaire ou lamellaire, sphérique, oblongue ou toute forme approchante), quelle que soit leur forme cristallographique (par exemple de type feuillet, cubique, hexagonale ou orthorhombique).

Les particules de la phase particulaire peuvent notamment être enrobées par au moins un composé siliconé tels que des polydiméthylsiloxanes et/ou par des polymères, notamment des polyéthylènes et/ou au moins un composé fluoré et/ou au moins un amino acide.

Par pigments, il faut comprendre des particules blanches ou colorées, minérales ou organiques, insolubles dans le milieu, destinées à colorer et/ou opacifier la composition.

Les pigments peuvent être présents à une teneur allant de 0,1 à 30%, de préférence de 0,5 à 15 %, en poids par rapport au poids total de la phase particulaire. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes, les nanozincs, le bleu ferrique. Parmi les pigments organiques, on peut citer le noir de carbone, et les laques comme les sels de calcium, de baryum, d'aluminium ou de zirconium, de colorants acides tels que les colorants halogéno-acides, azoïques ou anthraquinoniques.

Les pigments peuvent notamment être enrobés par des composés siliconés tels que des polydiméthylsiloxanes et/ou par des polymères, notamment des polyéthylènes. On peut ainsi citer les « oxydes SI » qui sont des pigments enrobés polyméthylhydrogénosiloxane vendus par la société Myioshi.

Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage.

Les charges peuvent être présentes dans la phase particulaire à une teneur allant de 70 à 100 %, en poids par rapport au poids total de la phase particulaire. Elles peuvent être minérales ou de synthèse. On peut citer le talc, le mica, la silice, le kaolin, les poudres de Nylon, de poly-β-alanine et de polyéthylène, le Téflon, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques telles que l'Expancel (Nobel Industrie), les microéponges comme le Polytrap (Dow Corning), les séricites, les argiles et les microbilles de résine de méthylsesquioxane (Tospearls de la Société TOSHIBA, par exemple), les oxydes de zinc et de titane, les oxydes de zirconium et de cérium, le carbonate de calcium précipité, le carbonate et l'hydrocarbonate de magnésium, l'hydroxyapatite, les microsphères de silice creuses (Silica Beads de la Société MAPRECOS), les microcapsules de verre et de céramique ; les savons métalliques dérivés d'acides organiques carboxyliques ayant de 8 à 22 atomes de carbone, de préférence de 12 à 18 atomes de carbone, par molécule, tels que par exemple le stéarate de zinc, de magnésium ou de lithium, le laurate de zinc, le myristate de magnésium, et leurs mélanges.

Par nacres, il faut comprendre des particules irisées qui réfléchissent la lumière.

Parmi les nacres envisageables, on peut citer la nacre naturelle, le mica recouvert d'oxyde de titane, d'oxyde de fer, d'hydroxyde d'aluminium, d'hydroxyde de magnésium, de silice de pigment naturel ou d'oxychlorure de bismuth ainsi que le mica titane coloré. Les compositions selon l'invention peuvent également comprendre des paillettes.

De préférence, la phase particulaire est présente dans les compositions selon l'invention à une teneur comprise entre 70 et 97%, en poids par rapport au poids total de la composition.

La composition selon l'invention a de préférence la formulation suivante, en poids par rapport au poids total de la composition :
- De 0,3 à 10%, de préférence de 0,6 à 6%, d'agent gélifiant de type élastomère de silicone à propriété tensioactive (matière active de polymère),
- De 50 à 98 %, de préférence de 70 et 97 %, de phase particulaire, et
- De 1,5 à 49,7 %, de préférence de 1,5 à 40 %, de façon encore plus préférée de 1,5 à 30 %, en poids par rapport au poids total de la composition, d'une phase grasse qui comprend de 0,6 à 40 %, de préférence de 0,6 à 30%, de façon encore plus préférée de 0,6 à 20%, d'une phase grasse liquide, le complément à 100% poids en phase grasse étant au moins un élément choisi parmi les corps gras pâteux, solides ou semi-solides.

Selon un autre mode de réalisation de l'invention, la composition peut comprendre de l'eau. Cette eau peut former la phase aqueuse d'une émulsion réalisée à partir de l'agent gélifiant qui est de préférence un organopolysiloxane polyoxyalkyléné, dans le but de former une émulsion à phase continue lipophile de préférence à phase continue à base d'huile.

La composition de l'invention peut donc comprendre un composé hydrophile choisi parmi l'eau, les solvants miscibles à l'eau en toute proportion comme les polyols (glycérol, diglycérine, éthylène glycol), les monoalcools inférieurs en C₂ à C₅, l'acétone, et la dicétone, et leurs mélanges. De préférence, la teneur en composé hydrophile peut représenter de 0,1 à 20%, de préférence de 0,2 à 15 %, en poids par rapport au poids total de la composition.

Avantageusement, dans un tel mode de réalisation, la composition permet d'apporter de la fraîcheur tout en préservant le confort à l'application.

D'autre part, il est aussi possible de réaliser une composition selon l'invention en utilisant comme liant une émulsion à phase continue hydrophile, de préférence à phase continue aqueuse. Dans ce cas est présent un autre tensioactif hydrophile que l'agent gélifiant, c'est-à-dire de HLB généralement supérieur à 8, par exemple, à concentration généralement supérieure à celle de l'agent gélifiant.

Avantageusement, la composition de l'invention peut contenir au moins un gélifiant de phase aqueuse, à savoir un composé capable de donner l'aspect d'un gel au liant et de l'épaissir. Parmi les gélifiants de phase aqueuse utilisables selon l'invention, on peut citer : les gélifiants cellulosiques hydrosolubles tels que l'hydroxyéthylcellulose, la méthylcellulose, l'hydroxypropylcellulose, la carboxyéthylcellulose et la carboxyméthylcellulose ; la gomme de guar ; la gomme de guar quaternisée ; les gommes de guar non-ioniques comprenant des groupements hydroxyalkyle en C₁-C₆ ; les gommes de xanthane, de caroube, de scléroglucane, de gellane, de karaya ; les alginates, la maltodextrine, l'amidon et ses dérivés, l'acide hyaluronique et ses sels ; les argiles et notamment les montmorillonites, les hectorites ou bentones, les laponites ; les polymères à groupement carboxylique comme les acides polyacryliques réticulés au moins partiellement neutralisés tels que les «Carbopol » ou «Carbomer» de la Société Goodrich (Carbomer 980 par exemple neutralisé par de la triéthanolamine -TEA en abréviation-) ; les polymères poly(métha)crylates de glycéryle ; la polyvinylpyrrolidone ; l'alcool polyvinylique ; les polymères et les copolymères réticulés d'acrylamide ; les homopolymères réticulés de chlorure de méthacryloyloxyéthyltriméthylammonium ; les polyuréthanes associatifs et leurs mélanges.

Selon l'invention, le gélifiant de phase aqueuse est de préférence choisi parmi la gomme de xanthane, les argiles (telles que la bentone ou la laponite), les polyuréthanes associatifs, les épaississants cellulosiques, notamment l'hydroxyéthyl cellulose, les acides polyacryliques réticulés au moins partiellement neutralisés, et leurs mélanges.

La composition de l'invention peut comprendre, en outre, tout additif usuellement utilisé dans le domaine concerné, qu'il soit hydrosoluble ou non, stable ou non en dehors de la composition. Ainsi l'additif peut être choisi dans le groupe formé parles colorants hydrosolubles ou liposolubles, les antioxydants, les huiles essentielles, les conservateurs, les actifs cosmétiques ou dermatologiques tels que les actifs instables en milieu oxydant, les actifs hydrophiles ou lipophiles, les polymères liposolubles notamment hydrocarbonés tels que les polyalkylènes, les gélifiants de phase grasse, les solvants, les absorbeurs d'odeur, les parfums, les électrolytes comme les sels inorganiques divalents ou monovalents (NaCI, MgCl₂, MgSO₄). les conservateurs, les antioxydants, les matières colorantes, et leurs mélanges, dans la mesure où l'additif ne déstabilise pas l'actif présent dans la composition qu'est l'agent gélifiant. Cet additif, selon sa nature, peut être introduit dans la phase grasse liquide ou dans la phase aqueuse éventuelle

Cet additif peut être présent dans la composition selon les quantités habituellement utilisées, par exemple en quantité de 0 à 20%, de préférence de 0,01 à 20%, de façon plus préférée de 0,1 à 10%, en poids par rapport au poids total de la composition. Pour les électrolytes, on utilise, de façon usuelle mais non obligatoire, au moins 30 à 60 milliosmoles ou mOsmol.

Par exemple, on peut citer notamment comme actifs instables en milieu oxydant, les vitamines et notamment l'acide ascorbique (vitamine C) et ses dérivés, notamment ses dérivés glycosylés et phosphatés, et ses esters comme l'acétate, le palmitate et le propionate d'ascorbyle, le rétinol (vitamine A) et ses dérivés, notamment ses esters comme l'acétate, le palmitate et le propionate de rétinol ; l'urée ; la rutine ; les enzymes telles que la lipase, la protéase, la phospholipase et les cellulases ; les extraits naturels tels que le thé vert, l'extrait de mélisse, l'extrait de thym, les oligomères procyannidoliques (OPC) tels que l'OPC d'aubépine, l'OPC de pin et l'OPC de raisin ; certains acides tels que l'acide kojique, l'acide caféique, l'acide rétinoique et ses dérivés, l'acide benzène 1,4-di-(3-méthylidène 10-camphosulfonique) ; les caroténoïdes tels que les carotènes comme par exemple les α-, β- et γ-carotènes, le β,ϕ-carotène, le ξ-carotène, le β,λ-carotène, le lycopène (ψ,ψ-carotène) ; les acides gras polyinsaturés tels que l'acide gamma-linolénique, et leurs mélanges. Il peut s'agir également de tous les composés naturels ou synthétiques pouvant contenir les actifs indiqués ci-dessus, en particulier les extraits végétaux, et plus spécialement les extraits de fruits.

Bien entendu l'homme du métier veillera à choisir les éventuels additifs complémentaires et/ou leur quantité de telle manière que les propriétés avantageuses de la composition selon l'invention ne soient pas, ou substantiellement pas, altérées par l'adjonction envisagée. En particulier, ces additifs ne devront pas nuire à l'homogénéité, la stabilité, le confort, la matité et la fraîcheur de la composition.

La composition selon l'invention se présente généralement sous la forme d'une poudre, par exemple libre, compactée ou pressée. La forme finale de présentation de la poudre en tant que produit fini prêt à l'utilisation peut être variée telle qu'une forme de dôme ou de sphère. Toute géométrie de présentation entre dans le cadre de la présente invention. Dans le cas d'une poudre libre par exemple, le liant peut représenter jusqu'à 15% en poids, par rapport au poids total de la composition, de préférence de 1 à 5% en poids. Pour une poudre compacte, la teneur en liant peut représenter de 1 à 30% en poids, de préférence de 3 à 20% en poids, par rapport au poids total de la composition.

Les compositions selon l'invention peuvent être préparées selon les méthodes connues de préparation des poudres cosmétiques, en tenant compte éventuellement de la volatilité de la ou des huiles utilisées ainsi qu'il est connu de l'homme du métier.

Les exemples de compositions ci-après sont donnés à titre illustratif de l'invention et sans caractère limitatif.

### Exemple 1 selon l'invention :

On réalise une poudre selon l'invention comprenant les constituants exprimés ci-après, en % poids par rapport au poids total de la composition. A cet effet, l'ensemble des composés, à l'exception du liant, est mélangé dans un appareil Baker Perkins. Le liant est ajouté sous agitation dans un broyeur type Hosokawa Alpine.

| | |
|---|---|
| Talc | 83 % |
| Poudre de nylon-12 | 10% |
| Nanotitane | 1% |
| Pigment oxyde de fer | 3% |
| Elastomère de silicone polyoxyalkyléné* | 3% en MP brute |
| Conservateur | qs |

| | |
|---|---|
| *: de nom commercial KSG21 de la Société Shin Etsu | |

### Exemple 2 comparatif :

On réalise une poudre comparative, ne faisant pas partie de l'invention, comprenant les constituants exprimés ci-après, en % poids par rapport au poids total de la composition, avec le même mode opératoire qu'à l'exemple 1 .

| | |
|---|---|
| Talc | 83% |
| Poudre de nylon-12 | 10% |
| Nanotitane | 1% |
| Pigment oxyde de fer | 3% |
| Liant silicone | 3% |
| Conservateur | qs |

Le liant silicone est un mélange des trois constituants suivants :
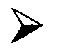 Abil wax 9801 de Goldschmidt : poly methylcetyl dimethylsiloxane (PM : 900-viscosité 15-25 cSt),
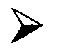 DC Fluid 953 de Dow Corning, mélange de résine trimethylsiloxysilicate et de PDMS (33/67, viscosité 700cSt),
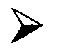 DC fluid 200 de Dow Corning, PDMS (viscosité 10 cSt).

### Evaluation

Les deux compositions des exemples 1 et 2 ont été évaluées par un panel de six consommatrices, en comparatif simultané par demi-visage.

L'évaluation est établie en fonction de quatre critères, à savoir
(1) matité à t = 0, où t est le temps (au contact avec la peau, au moment de l'application)
(2) tenue de la matité à t= 4heures
(3) homogénéité de la couleur à t=0
(4) tenue de la couleur à t=4 heures
Une échelle de graduation arbitraire allant de 0 à 18,5 à été proposée à chaque consommatrice où 0 est la borne minimale et 18,5, la borne maximale de la propriété évaluée.

Les résultats sont donnés dans le tableau suivant.

| | Poudre selon l'invention (exemple 1) | poudre comparative de l'exemple 2 | Différence |
|---|---|---|---|
| (1) matité | 14,5 | 13,1 | 1,4 |
| (2) tenue de la matité | 13,6 | 11,7 | 1,9 |
| (3) homogénéité de la couleur | 14,6 | 14,3 | 0,3 |
| (4) tenue de la couleur | 17,0 | 13,7 | 0,3 |

Il apparaît que la poudre la plus mate (critère 1) et la plus homogène en terme de couleur (critère 3) est celle de l'exemple 1 renfermant de l' élastomère de silicone. 4 heures après l'application, on voit que la poudre selon l'invention reste plus mate que la poudre de l'exemple comparatif 2, avec une différence encore plus importante qu'à l'application (critère 1). De plus, en terme d'homogénéité de couleur, on voit que le résultat est toujours aussi avantageux pour la poudre selon l'invention à t=4 heures (critère 4) qu'à l'application (critère 3) que pour la poudre comparative.

## Revendications

1. Composition cosmétique pulvérulente comprenant au moins une phase particulaire et au moins une phase grasse, **caractérisée en ce qu'**elle comprend en outre au moins un agent gélifiant de type élastomère de silicone à propriété tensioactive, et la phase particulaire étant présente en une teneur comprise entre 50 et 98 % en poids, par rapport au poids total de la composition.

2. Composition selon l'une des revendications précédentes telle que l'agent gélifiant est un organopolysiloxane élastomérique solide réticulé sous forme de particules et comportant au moins un groupement oxyalkyléné ayant de 2 à 4 atomes de carbone.

3. Composition selon la revendication 2 telle que l'organopolysiloxane élastomérique comporte au moins un groupement oxyéthyléné.

4. Composition selon l'une des revendications 2 ou 3 telle que l'organopolysiloxane élatomérique ne comporte que des groupements oxyéthylénés comme groupement oxyalkylénés ayant de 2 à 4 atomes de carbone.

5. Composition selon l'une des revendications 2 à 4 telle que l'organopolysiloxane élastomérique est obtenu par réaction d'addition et de réticulation en milieu non aqueux, en présence d'un catalyseur du type platine, d'au moins :
- un premier organopolysiloxane (i) ayant au moins deux groupements vinyliques en position α-ω de la chaîne siliconée par molécule ; et
- d'un second organopolysiloxane (ii) ayant au moins un atome d'hydrogène lié à un atome de silicium par molécule et au moins un groupement oxyalkyléné ayant de 2 à 4 atomes de carbone et notamment oxyéthylèné.

6. Composition selon la revendication 5 telle que le premier organopolysiloxane (i) est choisi parmi les polydiméthylsiloxanes.

7. Composition selon l'une des revendications 5 ou 6 telle que le premier organopolysiloxane (i) est un α-ω-diméthylvinyl polydiméthylsiloxane.

8. Composition selon l'une des revendications 5 à 7 telle que le second organopolysiloxane (ii) est choisi parmi les polydiméthylsiloxanes comportant un ou plusieurs atomes d'hydrogène et un ou plusieurs groupements oxyalkylénés, liés à un atome de silicium via un radical alkylène ayant de 1 à 22 atomes de carbone.

9. Composition selon l'une des revendications 2 à 8 telle que les particules d'organopolysiloxanes ont une taille allant de 0,1 à 500 µm, de préférence de 3 à 200 µm, et de façon encore plus préférée de 3 à 50 µm.

10. Composition selon l'une quelconque des revendications précédentes, **caractérisée par le fait que** la phase grasse comprend au moins une huile.

11. Composition selon la revendication précédente telle que l'huile est une huile siliconée.

12. Composition selon la revendication 10 ou 11, **caractérisée par le fait que** l'huile est une huile volatile.

13. Composition cosmétique selon l'une des revendications précédentes telle qu'elle comprend entre 70 et 97 %, de phase particulaire, en poids par rapport au poids total de la composition.

14. Composition selon l'une des revendications précédentes telle qu'elle comprend de 0,3 % à 10 % en poids, de préférence de 0,6 à 6 % en poids, d'agent gélifiant, par rapport au poids total de la composition.

15. Composition selon l'une des revendications précédentes telle que la phase grasse représente généralement de 1,5 à 49,7 % en poids, de préférence de 1,5 à 40 % en poids, de façon encore plus préférée de 1,5 à 30 % en poids, par rapport au poids total de la composition.

16. Composition selon l'une des revendications précédentes, **caractérisée en ce que** la phase particulaire comprend des pigments et/ou des nacres et/ou des charges et/ou des paillettes et/ou leurs mélanges.

17. Composition selon la revendication 16 telle que les pigments sont choisis parmi les dioxydes de titane, de zirconium ou de cérium, les oxydes de zinc, de fer ou de chrome, les nanotitanes, les nanozincs, le bleu ferrique, le noir de carbone, les sels de calcium, de baryum, d'aluminium ou de zirconium, les colorants halogéno-acides, azoïques ou anthraquinoniques, les pigments enrobés par des composés siliconés tels que des polydiméthylsiloxanes et/ou par des polymères.

18. Composition selon la revendication 16 ou 17 telle que les pigments sont présents à une teneur allant de 0,1 à 30%, de préférence de 0,5 à 15 %, en poids par rapport au poids total de la phase particulaire.

19. Composition selon la revendication 16 telle que les charges sont choisies parmi le talc, le mica, la silice, le kaolin, les poudres de Nylon, de poly-β-alanine et de polyéthylène, la lauroyl-lysine, l'amidon, le nitrure de bore, l'oxychlorure de bismuth, les poudres de polymères de tétrafluoroéthylène, les poudres de polyméthylméthacrylate, les poudres de polyuréthanne, les poudres de polystyrène, les poudres de polyester, les microsphères creuses synthétiques, les microéponges, les microbilles de résine de méthylsesquioxane, les oxydes de zinc et de titane, les oxydes de zirconium et de cérium, le carbonate de calcium précipité, le carbonate et l'hydrocarbonate de magnésium, l'hydroxyapatite, les microsphères de silice creuses, les microcapsules de verre et de céramique, les savons métalliques dérivés d'acides organiques carboxyliques ayant de 8 à 22 atomes de carbone, de préférence de 12 à 18 atomes de carbone, par molécule, et leurs mélanges.

20. Composition selon l'une des revendications 16 et 19 telle que les charges sont présentes à une teneur allant de 70 à 100%, en poids, par rapport au poids total de la phase particulaire.

21. Composition selon la revendication 16 telle que la nacre est choisie parmi la nacre naturelle, le mica recouvert d'oxyde de titane, d'oxyde de fer, d'hydroxyde d'aluminium, d'hydroxyde de magnésium, de silice de pigment naturel ou d'oxychlorure de bismuth ainsi que le mica titane coloré.

22. Composition selon l'une des revendications précédentes de formulation suivante, en poids par rapport au poids total de la composition :
• De 0,3 à 10%, de préférence de 0,6 à 6%, d'agent gélifiant de type élastomère de silicone à propriété tensioactive (matière active de polymère),
• De 50 à 98 %, de préférence de 70 et 97 %, de phase particulaire, et
• De 1,5 à 49,7 %, de préférence de 1,5 à 40 %, de façon encore plus préférée de 1,5 à 30 %, en poids par rapport au poids total de la composition, d'une phase grasse qui comprend de 0,6 à 40 %, de préférence de 0,6 à 30%, de façon encore plus préférée de 0,6 à 20%, d'une phase grasse liquide, le complément à 100% poids en phase grasse étant au moins un élément choisi parmi les corps gras pâteux, solides ou semi-solides.

23. Composition selon l'une des revendications précédentes telle qu'elle comprend un composé hydrophile choisi parmi l'eau, les solvants miscibles à l'eau, et leurs mélanges.

24. Composition selon la revendication précédente telle que le composé hydrophile représente de 0,1 à 20% en poids, de préférence de 0,2 à 15 % en poids, par rapport au poids total de la composition.

25. Composition selon la revendication 23 ou 24 telle qu'elle contient au moins un gélifiant de phase aqueuse choisi parmi l'hydroxyéthylcellulose, la méthylcellulose, l'hydroxypropylcellulose, la carboxyéthylcellulose et la carboxyméthylcellulose ; la gomme de guar ; la gomme de guar quaternisée ; les gommes de guar non-ioniques comprenant des groupements hydroxyalkyle en C₁-C₆ ; les gommes de xanthane, de caroube, de scléroglucane, de gellane, de karaya ; les alginates, la maltodextrine, l'amidon et ses dérivés, l'acide hyaluronique et ses sels ; les montmorillonites, les hectorites ou bentones, les laponites ; les acides polyacryliques réticulés au moins partiellement neutralisés, les polymères poly(métha)crylates de glycéryle ; la polyvinylpyrrolidone; l'alcool polyvinylique ; les polymères et les copolymères réticulés d'acrylamide ; les homopolymères réticulés de chlorure de méthacryloyloxyéthyltriméthylammonium; les polyuréthanes associatifs et leurs mélanges.

26. Composition selon la revendication 23 telle que le gélifiant de phase aqueuse est choisi parmi la gomme de xanthane, la bentone, la laponite, les polyuréthanes associatifs, l'hydroxyéthyl cellulose, les acides polyacryliques réticulés au moins partiellement neutralisés, et leurs mélanges.

27. Composition selon l'une des revendications précédentes telle qu'elle comprend en outre un additif choisi dans le groupe formé par les colorants hydrosolubles ou liposolubles, les antioxydants, les huiles essentielles, les conservateurs, les actifs cosmétiques ou dermatologiques, les polyalkylènes, les gélifiants de phase grasse, les solvants, les absorbeurs d'odeur, les parfums, les sels inorganiques divalents ou monovalents, les conservateurs, les antioxydants, les matières colorantes, et leurs mélanges.

28. Composition selon l'une quelconque des revendications précédentes, **caractérisée par le fait qu'**elle est une composition de maquillage.

29. Composition selon l'une des revendications précédentes telle qu'elle se présente sous la forme de fard à paupières, de fard à joues, de fard à sourcils, de poudre du visage et/ou du corps, de produit anticernes, de produit matifiant ou de produit de maquillage du corps.

30. Procédé cosmétique de maquillage ou de soin des matières kératiniques des êtres humains, en particulier de la peau du visage et du corps, comprenant l'application sur les matières kératiniques d'une composition telle que définie à l'une des revendications précédentes.

31. Utilisation d'au moins un agent gélifiant de type élastomère de silicone à propriété tensioactive dans une composition cosmétique pulvérulente, dans le but d'apporter de la matité aux matières kératiniques sur lesquelles est appliquée ladite composition.

32. Utilisation d'au moins un agent gélifiant de type élastomère de silicone à propriété tensioactive, dans une composition cosmétique pulvérulente, dans le but d'améliorer le développement de la couleur au sein de ladite composition.

33. Utilisation d'au moins d'un agent gélifiant de type élastomère de silicone à propriété tensioactive, dans une composition ou pour la fabrication d'une composition de soin ou de maquillage pour matifier la peau ou les lèvres et/ou camoufler les imperfections de la peau et/ou des lèvres.
